(19) Europäisches Patentamt
European Patent Office
Office européen des brevets

(11) Publication number: **0 516 895 A1**

(12) **EUROPEAN PATENT APPLICATION**

(21) Application number: **91201375.2**

(22) Date of filing: **04.06.91**

(51) Int. Cl.⁵: **G05B 13/04**, G05B 21/02, C12M 1/00

(43) Date of publication of application: **09.12.92 Bulletin 92/50**

(84) Designated Contracting States: **AT BE CH DE DK ES FR GB GR IT LI LU NL SE**

(71) Applicant: **UNILEVER N.V.**
**Weena 455**
**NL-3013 AL Rotterdam(NL)**

(72) Inventor: **Reyman, Greg Unilever Research Laboratorium**
**Vlaardingen Olivier van Noortlaan 120**
**NL-3133 AT Vlaardingen(NL)**

(74) Representative: **Smulders, Theodorus A.H.J., Ir. et al**
**Vereenigde Octrooibureaux Nieuwe Parklaan 97**
**NL-2587 BN 's-Gravenhage(NL)**

# (54) A method for the adaptive stochastic control of a process.

(57) A method is described for the adaptive stochastic control of a process which can be modelled as comprising at least two discrete states, in this method at least one output variable of the process is analyzed and a current process state is determined by means of contextual pattern recognition of the output variable(s) making use of statistics, preferably Markov chain probabilities and probability density functions, the parameters of which are adaptively up-dated on basis of the measurement of the output variable(s); at least one action is carried out on at least one input variable of the process, which action is determined by means of receding horizon dynamic programming which minimizes a given cost function and which is based on the assumption that the state recognized by means of the contextual pattern recognition is the true state.

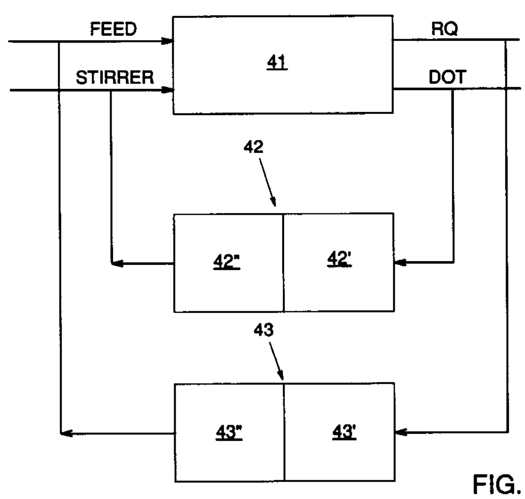


FIG. 4

EP 0 516 895 A1

The invention relates to a method for the adaptive stochastic control of a process in which at least one output variable of the process is analyzed and in which the results of this analysis are used to manipulate at least one input variable of the process by means of at least one action.

In recent decades a lot of attention has been paid to model and control of industrial processes. Most work and results are based on classical approach where first relevant process outputs and/or states and process inputs are determined and then the appropriate dynamic model is identified. Such a model in continuous or discrete time (frequency) domain is used as a basis for controller development.

The known models are either so-called black box models or theoretical models, which both, when used to develop a controller, in practice are not really suitable to match process control requirements.

The black box model is a linear model, while the process to be controlled by nature always is non-linear. This discrepancy automatically explains why the use of this kind of model as basis for a controller can not satisfy control requirements in practice.

The theoretical model needs a long development time and usually does not describe the reality exactly. There are no known applications in which such models have been used to develop practical controllers in process industry.

The invention aims to provide a process control method, which is more reliable than the methods used up to now.

The invention to this end is characterized in that the process is modelled as comprising at least two discrete states, that the history of the process is represented by a statistics, that a current process state is determined by means of contextual pattern recognition of the at least one output variable, that the at least one action to be carried out on the input variable is determined by means of receding horizon dynamic programming and that the statistics representing the process states is adaptively up-dated.

The method according to the invention is based on the observation that in reality a process operator is interested in reaching specific operational conditions and maintaining these or otherwise in switching to another operational regime. All deviations from this kind of operating regime should be reduced and transitions to new conditions should be as quick and smooth as possible. A given regime should be influenced neither by instrumentation noises nor by process disturbances. These kinds of operational conditions can be considered as discrete process states. Such states are for example various start-up, change-over and shut-down conditions, as well as steady-state conditions depending on product formulation and quality of raw materials, and all recognizable unwanted conditions to which the process can transit. For example, in edible oil hydrogenation such states can be: heating-up; adiabatic heating; maintaining operating conditions, mainly temperature and pressure; getting to the product specification zone and shut-down.

In crystallization of edible oils for example, which comprises various crystal modifications, it might be important to assure crystallization of a specific crystal modification. Crystallization regimes corresponding to forming of specific crystal modifications can be treated as specific process states.

In the example of a fermentation process of bakers' yeast, which will be considered in more detail in the following, such states can comprise: batch phase, i.e. growth on a glucose pulse while maintaining respiratory conditions, and fed-batch phase with three possible operating conditions: optimal growth on glucose, production of ethanol and ethanol consumption. The three last states are related to applied control strategy for substrate feed and aeration. Any of the above four fermentation states in accordance with an exemplatory embodiment of the invention are determined from current measurements of chosen process conditions and from the history of the process.

According to the invention, process dynamics, which is represented by transitions between at least two discrete states, is modelled using a Markov chain description. Markov chain transition probabilities can model the process transitions and in a natural way the process noise. For a more extensive description of the principle of a Markov chain reference is made to the article by R.D. Smallwood and E.J. Sondik, "Optimal control of partially observable processes over the finite horizon", Operations Research, Vol. 21, pp. 1071-1088, 1973.

The invention is based on the insight, that discrete state processes can be controlled by means of a control strategy that is based on the use of a Markov chain and on a static measurement description of the process. The use of a Markov chain is a well known technique in the field of operational control of machines etc., but never has been used for obtaining a controller for a process that is continuous in time.

In the following the invention shall be explained on the basis of the example of a three state process, with reference to the drawing in which:

Figure 1 shows the controlled Markov chain for a three state process;

Figure 2 shows possible density functions for such a process;

Figure 3 a,b,c show three possible states in a process for fed-batch fermentation of bakers' yeast;

Figure 4 shows schematically a control loop for the fed-batch fermentation of bakers' yeast;

Figure 5 shows some measurements obtained with the method according to the invention in the control loop of figure 4; and

Figure 6 shows possible density functions for the fed-batch fermentation of bakers' yeast.

Although the method according to the invention in the following will be described for a three state process and more particularly for the control of the fed-batch fermentation of bakers' yeast, it is emphasized that the invention with the same advantages can be used for the control of other three state processes, for processes with two or more than three states and even for the control of steady state processes, in which case for the method according to the invention the process is modelled by a three state controlled Markov chain, where the optimal state is at setpoint and two suboptimal states are above and below setpoint respectively.

First the principle of Markov chains as used in the control method according to the invention shall be explained.

Let $J_n = i$ be the state of the process on time n and let $k_n = k$ be the action that is carried out at time n, then the probability that the process on the time n+1 will be in the state $J_{n+1} = j$ due to the action $k_n$, can be described as

$$P\,(J_{n+1} = j \mid J_n = i, k_n = k) = pij^k(\phi),$$
with $0 \leqq pij^k(\phi) \leqq 1$.

Figure 1 shows by way of example the description of a three state process, i.e. j = 1, 2, 3, for three different actions k = 1, 2, 3 by means of a controlled Markov chain. The value of $pij^k$ depends on the value of the vector parameter $\phi$. In the following it shall be explained how this parameter $\phi$ can be determined in a relatively simple way.

Further, by way of example, during the process one variable x of the process is measured. However it is within the scope of the invention that more variables are measured and analyzed. The chance that the process at the time of the measurement of the variable x, which, in the case that more variables are measured, is multi-dimensional, is in the state j is given by:

$$f(x \mid j, \alpha),$$
with $0 \leqq f(x \mid j, \alpha) \leqq 1$.

In the exemplary case of a three state process, three different probability density functions $f_1$, $f_2$, $f_3$ of the measured process variable x are defined, which curves for example can have the form shown in figure 2. The configuration of these curves is determined by the value of $\alpha$, which is representative for the history of the process and which will be discussed in detail below.

Based on the above described principles of a controlled Markov chain and of a static measurement description, as given by probability density functions, a process in accordance to the invention is controlled as follows, by means of a contextual pattern recognition algorithm and by a receding horizon dynamic programming algorithm.

At the beginning of the control proces one has a first measured process variable $x_0$. By arbitrarily choosing a first estimate of the starting value $\alpha_o$, it is possible to determine the first three probability curves $f_1$, $f_2$ and $f_3$. Further, at the beginning of the control proces, one defines on basis of the practically always available knowledge of the process three initial probability values $p_1$, $p_2$ and $p_3$ which indicate the probability that the proces at that time is in the state 1, 2 or 3, with $p_1 + p_2 + p_3 = 1$.

On basis of $f_j$ and $p_j$ (j = 1, 2, 3) one defines :

$$d_o(j) = \frac{p_j * f_j\,(x_o \mid j, \alpha_o)}{\sum_{i=1}^{3} p_i * f_i\,(x_o \mid i, \alpha_o)} \qquad j = 1,\ 2,\ 3. \qquad \text{(Ia)}$$

This gives three statistics $d_o$ (1), $d_o$ (2) and $d_o$ (3), with $d_o(1) + d_o(2) + d_o(3) = 1$. With (I) the state of the process $i_o$ at t=0 is determined by:

$$i_o = \arg \{\max_j [d_0(j)]\} \qquad \text{(Ib)}$$

this means that it is assumed that the process is in that state j which has the highest statistics value $d_o(j)$.
At the following measuring moment n = 1, the statistics $d_o(1)$, $d_o(2)$ and $d_o(3)$ are known, further a process variable $x_1$ is measured, which makes it possible to calculate $f_j(x_1 \mid j,\alpha_1)$. Also known are $k_o$, the action that was carried out on the process at n = 0, and $i_o$, the recognized state at n = 0. On basis of all this information it is possible to recognize the state of the process $i_1$ at n = 1, which recognition takes into account the "history" of the process, which history of course is relatively short in the case of n = 1, by determining:

$i_1 = \arg\{\max_j [d_1(j)]\}$

with:

$$d_1(j) = \frac{[f_j(x_1 \mid j,\alpha_1)] * \sum_{i=1}^{3} p_{ij}^{k_o} * d_o(i)}{\sum_{\lambda=1}^{3}\{[f_\lambda(x_1 \mid \lambda,\alpha_1)] * \sum_{i=1}^{3} p_{i\lambda}^{k_o} * d_o(i)\}} \quad j = 1,2,3. \quad \text{(IIa)}$$

The denominator of (IIa) is necessary to normalize the values of $d_1(j)$ between 0 and 1. In the same manner as $d_1(j)$, all further values $d_n(j)$ can be determined with:

$$d_n(j) = \frac{[f_j(x_n \mid j,\alpha_n)] * \sum_{i=1}^{3} p_{ij}^{k_{n-1}} * d_{n-1}(i)}{\sum_{\lambda=1}^{3}\{[f_\lambda(x_n \mid \lambda,\alpha_n)] * \sum_{i=1}^{3} p_{i\lambda}^{k_{n-1}} * d_{n-1}(i)\}} \quad j = 1,2,3. \quad \text{(IIb)}$$

and from these values of $d_n(j)$ every time a corresponding recognized state $i_n$ can be determined with:

$i_n = \arg\{\max_j [d_n(j)]\}$,

the recognition of the state $i_n$ now being based on the last measured process variable $x_n$ and on the complete history of the process as represented by the previous calculated probabilities $d_{n-1}(j)$ and the Markov chain probabilities $p_{ij}$ for the previous action $k_{n-1}$. The foregoing forms the contextual pattern recognition algorithm. This algorithm also can be used for processes having q states, j = 1,2,3 ...q, in which case the variables i, j and $\lambda$ in formula (IIb) have the values 1,....q.

On basis of the recognized state $i_n$ a receding horizon controller can determine which action has to be taken at the time n as follows.

With the well known principle of cost functions, for a process under consideration it is possible to define in a suitably chosen way a cost factor $C_n(j,k)$, which means that when the process is in the state j and the action k is carried out, then the costs are $C_n$, which $C_n$ can be time dependent.

In the initial phase of the control process, n = 0, it is assumed that the actual state $j_o$ is the same as the recognized state $i_o$, which was determined with formula (Ib). In accordance with the invention the controller is arranged in such a way that it carries out that action $k^*_o$;
$k^*_o$ = 1, 2 or 3, for which the value $V_o(i_o,k^*_o) = C_o(i_o,k^*_o)$ is minimal. In general $k^*_n$ indicates the action k at time n which is the optimal action.

For n = 1, the history of the costs is taken into account and cost values $V_1(j,k)$ are determined:

$$V_1(j,k) = C_o(j,k) + \sum_{\lambda=1}^{3} V_o(\lambda,k^*_o) * p_{j\lambda}^{k}. \quad \text{(III)}$$

At time n = 1 the action $k^*_1$ is chosen for which the value $V_1(i_1,k)$ is minimal, $i_1$ being the the recognized state at this time.

When the process continues, each time n it is assumed, that the actual state $j_n$ is the same as the recognized state $i_n$ and, for determining the optimal action at a time n, new costs $V_n(j,k)$ are calculated taking into account the history of the costs. This is done by determining recursively values $V_n(j,k)$ for each possible action k in accordance with:

$$V_n(j,k) = C_{n-1}(j,k) + \sum_{\lambda=1}^{3} V_{n-1}(\lambda,k^*_{n-1}) * p_{j\lambda}^k \qquad (IV)$$

for each $n \geq 2$;

At the time n the controller chooses that action $k^*_n$ to be carried out next for which the value of $V_n(i_n,k)$ is minimal.

The determination of the minimal costs problem using (IV) is a well known dynamic programming algoritm, such as is described in the above mentioned article of R.D. Smallwood and E.J. Sondik, "Optimal control of partially observable processes over the finite horizon", Operations Research, Vol. 21, pp. 1071-1088, 1973.

Finally the control method according to the present invention involves a learning process to make the process control adaptive. In the learning process the most probable values of $\alpha$ and $\phi$ can be learned in the following way:

$p_{ij}^k(\phi)$ is determined by a maximum likelihood estimation of unknown Markov chain transitions probabilities, which is given by:

$$p_{ij}^k(\phi_n) = \frac{m_n(i,j,k)}{m_n(i,k)} \qquad (V)$$

In this formula $m_n(i,j,k)$ indicates the number of times that, at the time n, a state i (i = 1, 2 or 3) has been recognized, that the action k (k = 1, 2 or 3) is carried out and that this has as a result a new state j (j = 1, 2 or 3), for each combination of values for i, j and k. Further $m_n(i,k)$ indicates the total number of times that, at the time n, a state i has been recognized and the action k has been carried out for corresponding values of i and k as in the denominator of formula (V).

One can determine $f_j(x_n|j,\alpha_n)$ in the same manner as is described in the article of E. Parzen, "On estimation of a probability function and mode", Annals of Mathematical Statistics, Vol. 33, pp. 1065-1076, 1962.

In the more specific case of a three state process a more simple way of determining the probability curves $f_1$, $f_2$ and $f_3$ is possible at the hand of model dependent distribution curves. Such curves for example can be exponential, normal, lognormal etc. and the particular form of the type of curve(s) chosen for a specific process depends on initially available knowledge about the process. During the control of the process only the parameters of these distribution curves have to be estimated. Initial values for this parameters are tuning parameters at the start-up of the process control and during the control of the process these initial values are adaptively updated using well known statistical formulas for the specific model dependent distribution worked out in a recursive form.

Reference is made to figure 2. Assuming that a hypothetical process is a three state process and that $f_2$ is the curve having the maximum probability at the set-point s of the process. The curve $f_2$ then, on basis of initially available knowledge about the process, for example can be chosen to be a curve having a normal distribution around the set-point s with a allowed deviation $\delta$, and on the same basis the curves $f_1$ and $f_3$ can be chosen as being curves having also a normal distribution.

In the following the application of the process control method according to the invention shall be explained at the hand of the example of the control of the fed-batch fermentation of bakers' yeast.

Bakers' yeast fermentation can be performed in three different modes: continuous, batch, and fed-batch. The first of these operations is based on the continuous addition of substrate and simultaneous removal of products. In a batch operation no substrate is added to the initial charge nor is a product removed until the end of the process. In the fed-batch operation nutrients are fed either shot-wise or continuously during fermentation and the product is removed at the end of the process.

Fed-batch fermentation is very well suited for yeast production because cell growth and product formation are controlled by the concentration of the limiting substrate. The fed-batch mode provides an excellent means of regulating the nutrient feed rate to optimize the productivity whilst at the same time avoiding over and underfeeding of nutrients. Overfeeding of nutrients often leads to formation of undesired byproducts (e.g. ethanol) . Underfeeding of nutrients results in cell starvation and ethanol consumption resulting in reduced specific growth rate, thereby lowering the productivity of yeast biomass. The fed-batch yeast fermentation process has received wide attention for its ability to produce high density , high quality yeast biomass. There is considerable knowledge about steady state control of continuous yeast fermentation and batch fermentation which do not need sophisticated control strategies. However, fed-batch yeast fermentation requires the application of sophisticated control strategies to assure the optimal growth of yeast cells during the whole process. The closed loop control strategy is the best approach to ensure the optimal growth irrespective of possible disturbances of an environmental or physiological nature.

Depending on the level of complexity, fermentation models can be separated into structured and unstructured systems. Unstructured models usually describe the overall observed microbial response. No provision is made for changes in the internal composition of the cells when the metabolism is shifted. The structured models take into account the various cellular processes i.e activities of specific enzymes, which are important in the process. An advantage of structured models is their potential for predicting the relationship between the different metabolic processes and the effects of disturbances on the overall microbial process. In the example to be described a simple structured model was chosen.

The model originates from an article of B. Sonnleitner and O. Käppeli, "Growth of Saccharomyces cerevisiae is controlled by its respiration capacity: formulation and verification of a hypothesis", Biotechnology and Bioengineering, Vol. 28, pp. 927-937, 1986,and incorporates results described by A.P.J. Sweere, "Response of baking yeast to transient environmental conditions relevant to large-scale fermentation processes", Ph.D. Thesis, Drukkerij Elinkwijk, Utrecht 1988.

This simple structured model is based on the response of yeast to a glucose pulse during the fermentation under an assumption of limited respiratory capacity. Stoichiometric equations of the growth of bakers' yeast on glucose and/or ethanol are given below for oxidative, {1} and {3}, and reductive {2} metabolic pathways

$$\{1\}C_6H_{12}O_6 + aO_2 + b.nx[NH_3] \rightarrow bC_1H_{hx}O_{ox}N_{nx} + cCO_2 + dH_2O$$

glucose/oxygen/ammonia biomass/carbon dioxide/water

$$\{2\}C_6H_{12}O_6 + g.nx[NH_3] \rightarrow gC_1H_{hx}O_{ox}N_{nx} + hCO_2 + iH_2O + jC_2H_6O$$

glucose/ammonia biomass/carbon dioxide/water/ethanol

$$\{3\}C_2H_6O + kO_2 + p.nx[NH_3] \rightarrow pC_1H_{hx}O_{ox}N_{nx} + mCO_2 + nH_2O$$

ethanol/oxygen/ammonia biomass/carbon dioxide/water

Biomass has the assumed molecular formula $C_1H_{hx}O_{ox}N_{nx}$, where hx = 1.83, ox = 0.56 and nx = 0.17. The composition of biomass grown on glucose does not change significantly if ethanol is either co-consumed or produced. In the above equations a, b, c, d, g, h, i, j, k, m, n and p are stoichiometric parameters.

The limited respiration capacity of the yeast cells depends on the availability of dissolved oxygen in the broth and can be represented as a bottleneck.

Let us consider three different situations (cases) with respect to filling the above bottleneck with two possible substrates: glucose and ethanol.

Case 1. (see Figure 3a): If there is a mixture of glucose and ethanol in the medium and the glucose flux does not require maximal respiration, i.e. the glucose flux is subcritical, the specific rate of the oxidative pathway (1) will be determined by the glucose consumption rate. The glucose consumption rate follows Monod kinetics. In this case no ethanol is produced so the rate of pathway {2} will be zero. The residual respiration capacity can be utilized for ethanol growth. If there is no oxygen limitation then the ethanol consumption follows Monod kinetics. The priority of glucose consumption over ethanol consumption can be

formulated as inhibition by freely available glucose.

Case 2. (see Figure 3b): If the ethanol uptake is limited by the residual respiration capacity, then a part of the ethanol flux will not be metabolized.

Case 3. (see Figure 3c): On the other hand when the glucose flux is sufficiently high to exceed the respiration capacity, i.e. supracritical glucose flux, the respiration capacity of the yeast cells limits the specific rate of pathway {1} and all the oxygen will be used via this pathway. No oxygen is left for pathway {3} so the rate of this pathway is zero. The remainder of the glucose flux which could not be metabolized via pathway {1} will be utilized according to the reductive pathway {2}.

On the basis of the above analysis of metabolic peathways taking place during the fed-batch fermentation it can be concluded that the fermentation physiological conditions on the macro scale can be represented by a state given by one of the three above defined cases. Knowing the current state, it is then easy to determine a control strategy for a next fixed time horizon.

On basis of the above analysis three fermentation states are defined:

**state one** - ethanol production in the presence of excess glucose with respect to the limiting oxygen bottleneck,

**state two** - optimal growth on glucose, i.e., optimal fitting of the glucose flux into the oxygen bottleneck,

**state three** - consumption of ethanol by insufficient glucose flux to fill the whole oxygen bottleneck.

In practice it is possible at the moment to measure two important variables which give some information about yeast growth conditions and about the current fermentation state: dissolved oxygen tension (DOT) and respiratory quotient (RQ).

DOT can be measured continuously by a polarographic probe and practically a prefiltered value is available every 5 seconds. On the other hand RQ value is available from a mass spectrometer, in practice every 3 - 5 minutes. The above definition of three basic fermentation states yields by different rates of measurement of DOT and RQ a concept of multirate control of two separate loops: DOT is controlled by controlling the speed rate of a stirrer in case of a constant air flow rate, or by controlling the speedrate of the stirrer and parallelly the air flow rate, while RQ is controlled by controlling the glucose flow rate.

This concept is schematically depicted in Figure 4, in which block 41 depicts the process to be controlled, i.e. the fed-batch fermentation of bakers' yeast, with as input variables the glucose feed rate and the stirrer speed rate and as measured output signals the respiration quotient RQ and the dissolved oxygen tension DOT. The RQ is controlled by controlling the glucose flow rate by means of a control loop 42 comprising a contextual state recognition section 42' and a receding horizon dynamic programming section 42''. The DOT is controlled by controlling the stirrer by means of a control loop 43 comprising a contextual state recognition section 43' and a receding horizon dynamic programming section 43''.

From the above reasoning it follows that separate definitions of state for the DOT-loop and the RQ-loop are required. For the DOT loop j = 1 denotes state one, j = 2 state two and j = 3 state three. However for the RQ loop j = 1 denotes state three, j = 2 state two and j = 3 state one. This definition leads to uniform definition of control actions for both loops: k = 1 denotes incremental flow increase, k = 2 exponential incremental, a 'normal' increase and k = 3 flow decrease. A rough target trajectory has to be defined, e.g. a trajectory which would be followed in an open loop fermentation with no disturbances. Form this target trajectory and required initial conditions 'normal' exponentially growing increments for stirrer speed and glucose flow can be easily calculated. These increments are then implemented if the fermentation state is two, i.e. when there is optimal growth.

As is described in the above general desciption of the control method according to the invention, for the definition of the performance index costs $C_n$ should be given. For a desired time horizon of about ten scanning cycli, ten values should be defined. The most practical approach is to weight stronger undesired transitions, and assign weight equal to zero to transitions which are desired. This results in

$$C_n(j_n,k_n) = \alpha^{N-i}\, C(j,k)\ ,\ i = n,....,N$$

where n denotes a current time and $0 < \alpha < 1$. For e.g. state j = 1

$$C(1,k) = \begin{cases} 0 \text{ if } k = 1 \\ \gamma \text{ if } k = 2 \\ 1 \text{ if } k = 3 \end{cases} \qquad 0 < \gamma < 1$$

where for the state j = 1, k = 1 denotes required action, k = 2 'normal' action and k = 3 undesired action. For other states the C values are defined in the same way. In the above $\alpha$ and $\gamma$ are tuning parameters to be determined empirically.

Initial values of transition probabilities can be obtained from already available experimental data and schematically, without specific numerical values are shown in figure 1. To relate measurements with three defined states, probability density functions $f_1$, $f_2$ and $f_3$ are used, as shown in figure 6. From off-line analysis of the process data it appears to be logical to represent the measurement of a state two by a normal distribution with the mean equal to the required current setpoint m and deviation $\delta$ set equal to allowable deviation from this setpoint. The parameter $\delta$ is however free with respect to further tuning if required. Measurement of state one and state three are given by lognormal distributions. For the state one the density function is modified by a symmetric function equal to zero for negative arguments. Mean values and initial values of deviation are tuning parameters. These deviation values are estimated on-line during run of the controller.

Using the above values for the control method according to the invention during several simulation trials led to very good results. Results of control of RQ and DOT where RQ was measured every 5 minutes with 5% noise, and DOT was measured every 20 seconds with 5% noise are given in Figure 5 . After 15 hours the setpoint of RQ was step-changed.DoT setpoint was kept constant. After about 19 hours DOT is no more controlled on the required setpoint due to saturation of the stirrer speed. However, this has no effects on controlling RQ. This proves that the control method has excellent setpoint tracking and disturbance rejection proprieties and shows its robust performance in a very noisy process.

Another example of the application of the process control in accordance with the invention is in a fat crystallization process.

Edible fats are multi-component mixtures of triacylglycerols with different chain lengths. One of the basic characteristics of fats is the melting behaviour, which depends on the composition of the fat. The partial crystallization and subsequent solid/liquid separation of edible fats is a well known method to change the melting behaviour of the fat.

In the crystallization step two measurable process outputs are important, the amount of crystals i.e. the total mass, and the crystal size distribution, which is characterized by the parameters of a suitable model for size distributions. The variables with which these outputs can be influenced are the temperature or the temperature/time history and the stirring rate in the crystallization vessel.

A complicating factor in the process is the occurence of more than one crystal modification, i.e. different lattice structures, of the crystallizing fat components. Typical crystal modifications in fat crystallization processes are $\alpha$, $\beta$, and $\beta'$ modifications. Each modification has different kinetic constants for nucleation and growth, resulting in different solid phase contents and size distributions.

With the control in accordance with the invention it becomes possible to recognize the state of the process with respect to the crystal modification which is formed, based on the controller input temperature or temperature/time history and the parameters of the size distribution. It is also possible to predict the result of any action (temperature change and/or stirring rate) with respect to the modification and it becomes possible to determine the optimal process conditions for given setpoints for solid phase amount and size distribution.

For a typical crystallization process having two discrete process states, $\alpha$ and $\beta$ crystallization, by on-line measurement of parameters of crystal size distribution and the amount of crystal mass and at the hand of the transition probabilities, which are initially determined based on crystallization kinetics knowledge, and of probability density curves which are derived from crystal size distributions and crystallization kinetics knowledge, using the method according to the invention, one can control the temperature and stirrer speed increasing them or decreasing them incrementally in such a way that the desired product quality of the process in terms of crystal size distribution is obtained in a reliable way.

## Claims

1. A method for the adaptive stochastic control of a process in which at least one output variable of the process is analyzed and in which the results of this analysis are used to manipulate at least one input by means of at least one action, characterized in that the process is modelled as comprising at least two discrete states, that the history of the process is represented by a statistics, that a current process state being determined by means of contextual pattern recognition of the at least one output variable, that any action to be carried out on any input variable is determined by means of receding horizon dynamic programming and that the statistics representing the process states is adaptively updated.

**2.** A method in accordance with claim 1, characterized in that for the contextual pattern recognition use is made of Markov chain probabilities and of probability density functions, the parameters of which are up-dated on basis of the measurement of the output variable.

**3.** A method in accordance with claim 1 or 2, characterized in that any action is derived by a receding horizon dynamic programming which minimizes a given cost function and which is based on the assumption that the state recognized by means of the contextual pattern recognition is the true state.

**4.** A method in accordance with claim 1 and 2 , characterized in that a current process state i at a time n ($n \geqq 1$) of a process having j = 1,2 ... .q possible states is determined by means of the contextual pattern recognition formulae:

$i_n$ = arg {$\max_j$ [$d_n(j)$]}; where

$$d_n(j) = \frac{[f_j(x_n|j,\alpha_n)] * \sum_{i=1}^{q} p_{ij}{}^{k}{}_{n-1} * d_{n-1}(i)}{\sum_{\lambda=1}^{q}\{[f_\lambda(x_n|\lambda,\alpha_n)] * \sum_{i=1}^{q} p_{i\lambda}{}^{k}{}_{n-1} * d_{n-1}(i)\}} \quad j = 1,2,\ldots q.$$

in which:

$f_j(x(\ j,\alpha)$ with j = 1,2, ....,q, are probability density functions for the at least one output variable x, wherein $\alpha$ is a value determined by the history of the process; and $p_{ij}{}^{k}{}_{n-1}$ with i, j = 1,2,...,q, are Markov chain probabilities in which $k_{n-1}$ indicates the action carried out on an input variable at the time n-1.

**5.** A method in accordance with claim 1, 2 and 3 , characterized in that for an action $k_n$ to be carried out on an input variable the cost function V at a time n ( $n \geqq 1$) of a process having j = 1,2 ....q possible states is determined by:

$$V_n(j,k) = C_{n-1}(j,k) + \sum_{\lambda=1}^{q} V_{n-1}(\lambda,k^*{}_{n-1}) * p_{j\lambda}{}^{k};$$

in which $C_{n-1}(j,k)$ are the costs at the time n-1; and that the action $k^*_n$ is carried out for that $k_n$ for which the corresponding value of $V_n(j,k_n)$ is minimal.

**6.** A method in accordance with any of the claims 1 -5, wherein the process is a fed-batch fermentation of a yeast, in which a stirrer is used and an oxygen containing gas is introduced, characterized in that the output variable is the dissolved oxygen tension and the at least one input variable is selected from the group consisting of the stirrer speed rate and the flow rate of the oxygen containing gas.

**7.** A method in accordance with any of the claims 1 -5, characterized in that the process is a fed-batch fermentation of yeast, that the output variable is the respiration quotient and the input variable is the substrate feed rate.

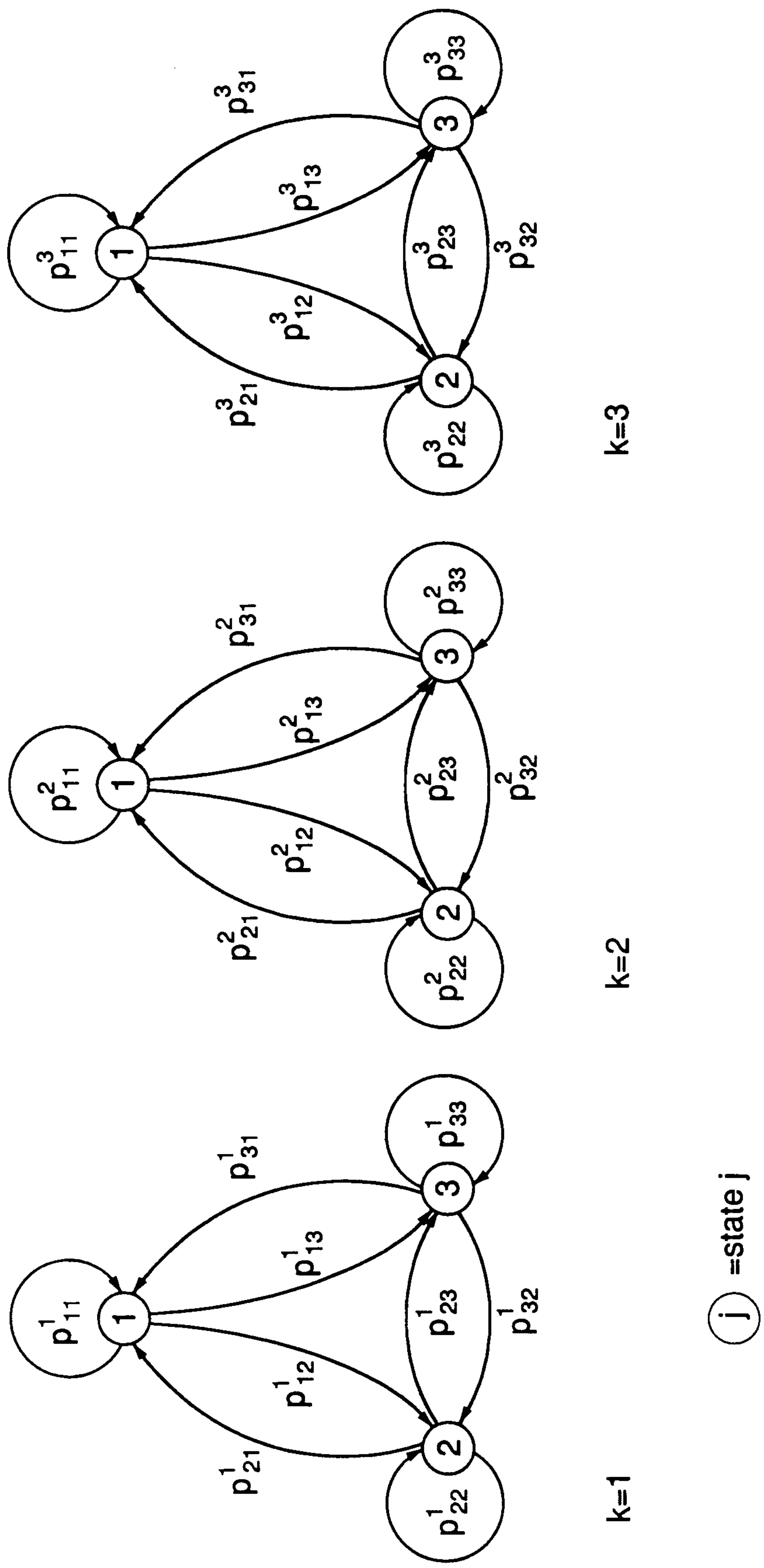


FIG. 1

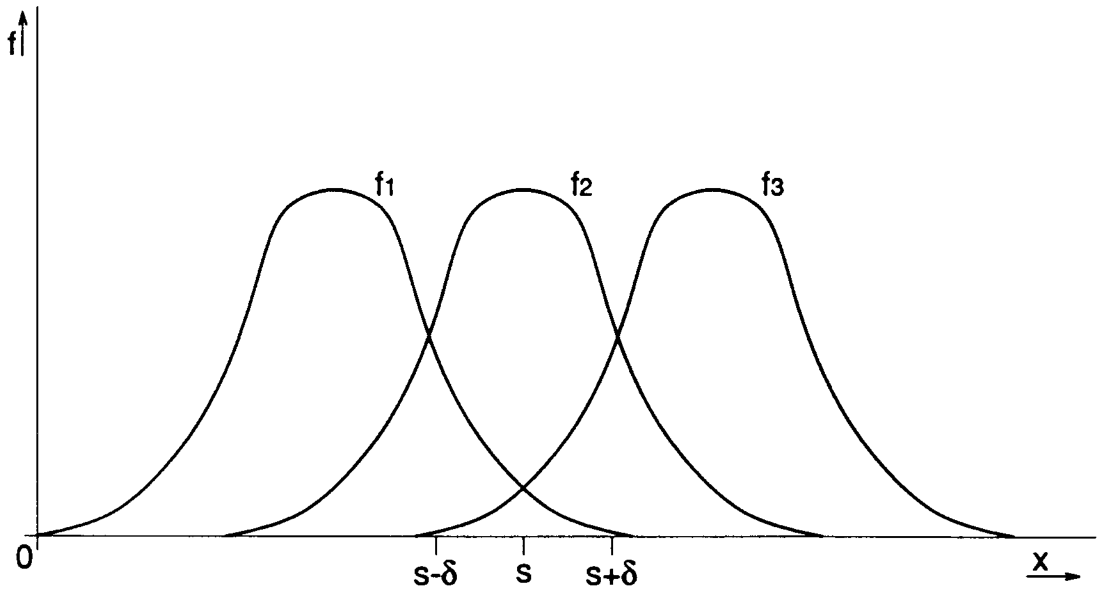


FIG. 2

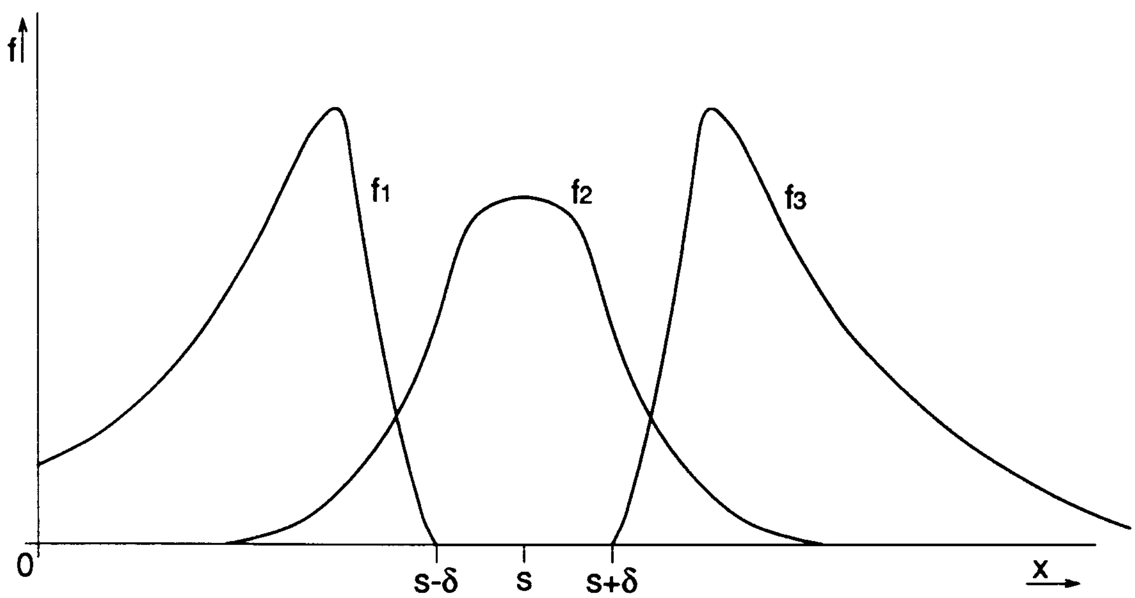


FIG. 6

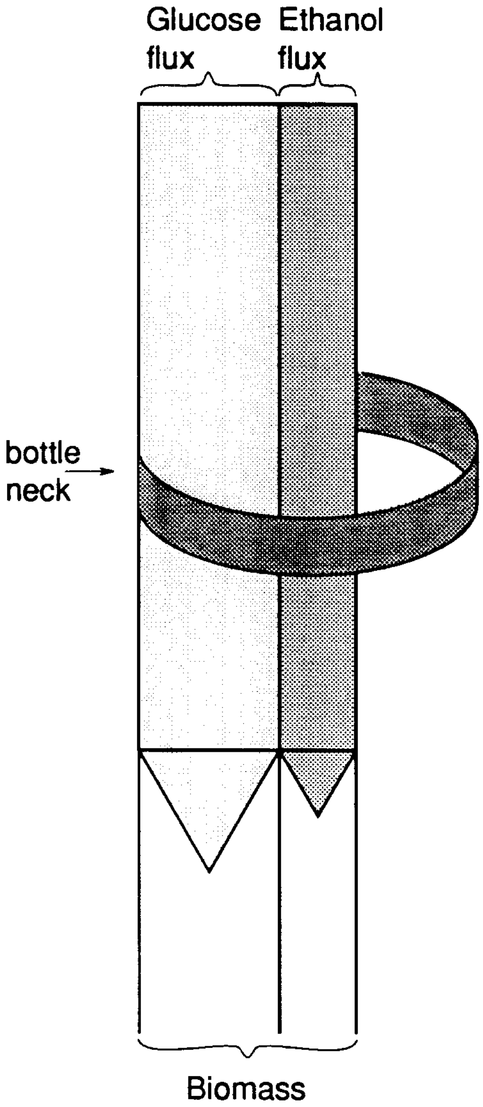
Glucose flux
Ethanol flux
bottle neck
Biomass

FIG. 3a

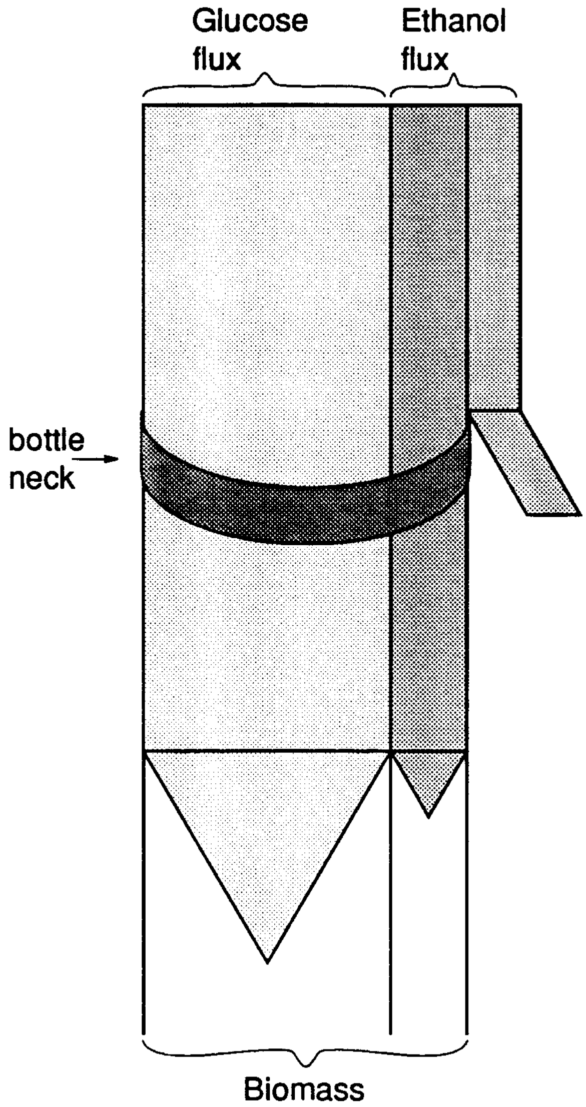
Glucose flux
Ethanol flux
bottle neck
Biomass

FIG. 3b

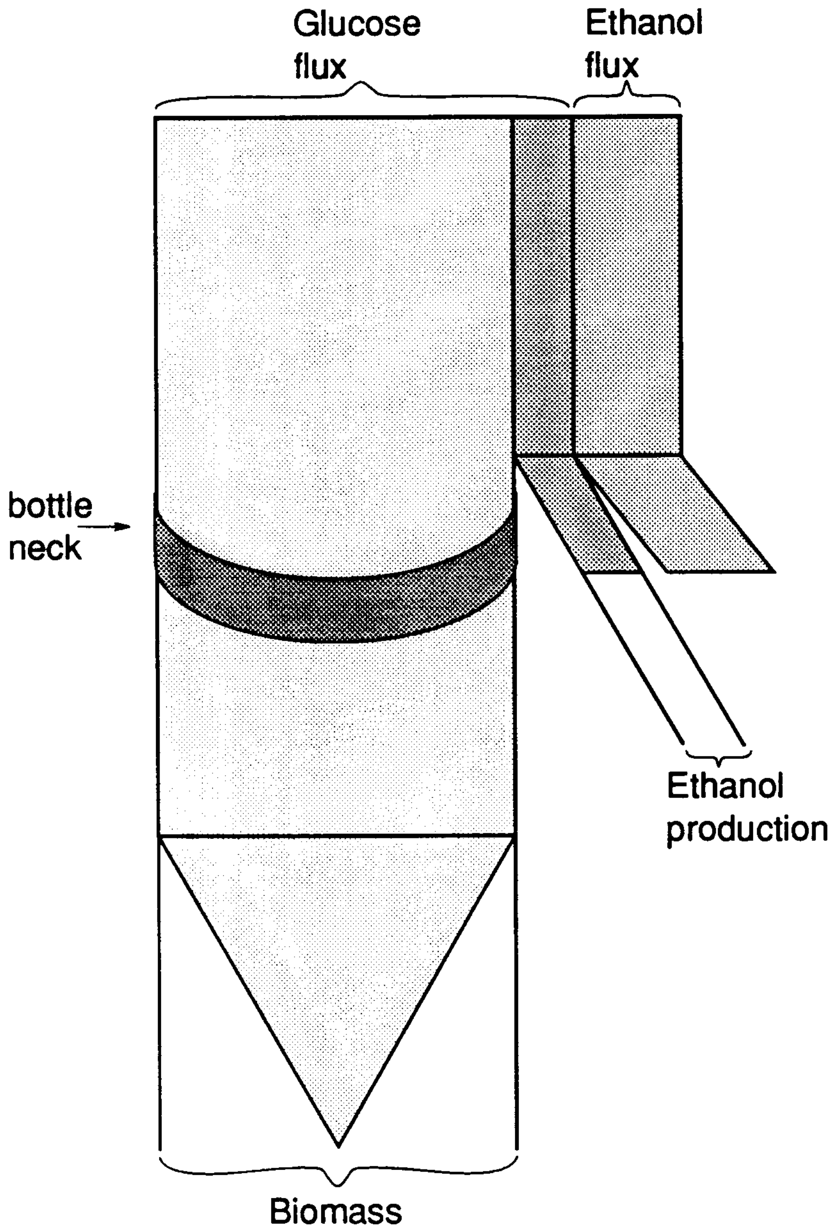
Glucose
flux
Ethanol
flux
bottle
neck
Ethanol
production
Biomass

FIG. 3c

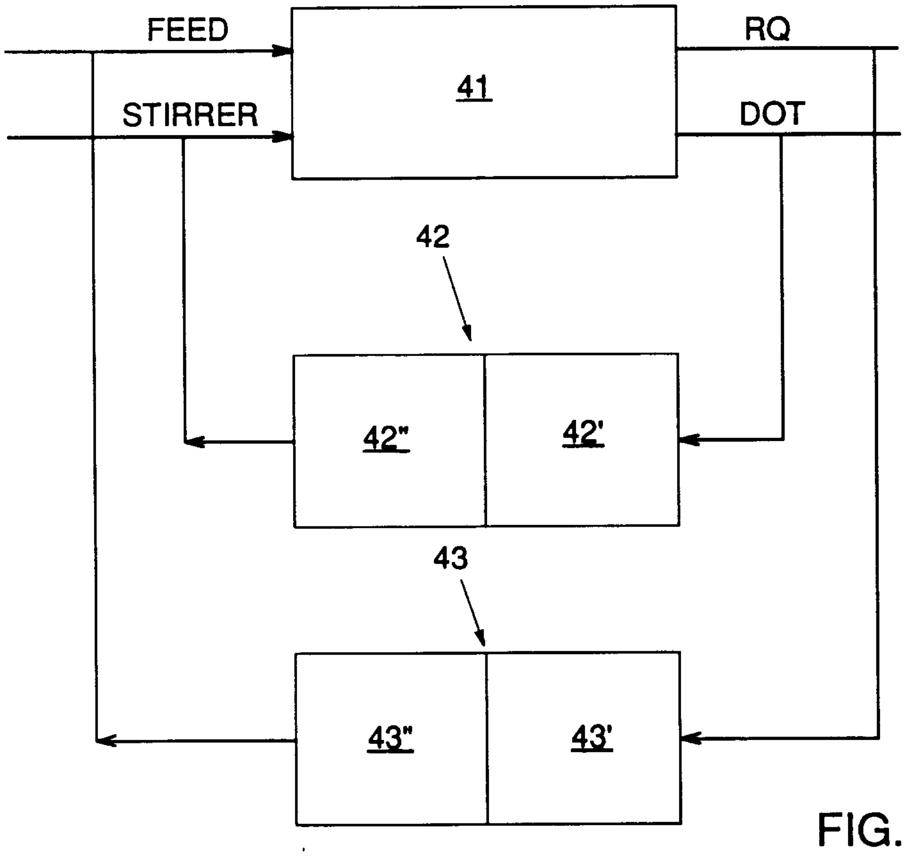
FEED
STIRRER
41
RQ
DOT
42
42"
42'
43
43"
43'

FIG. 4

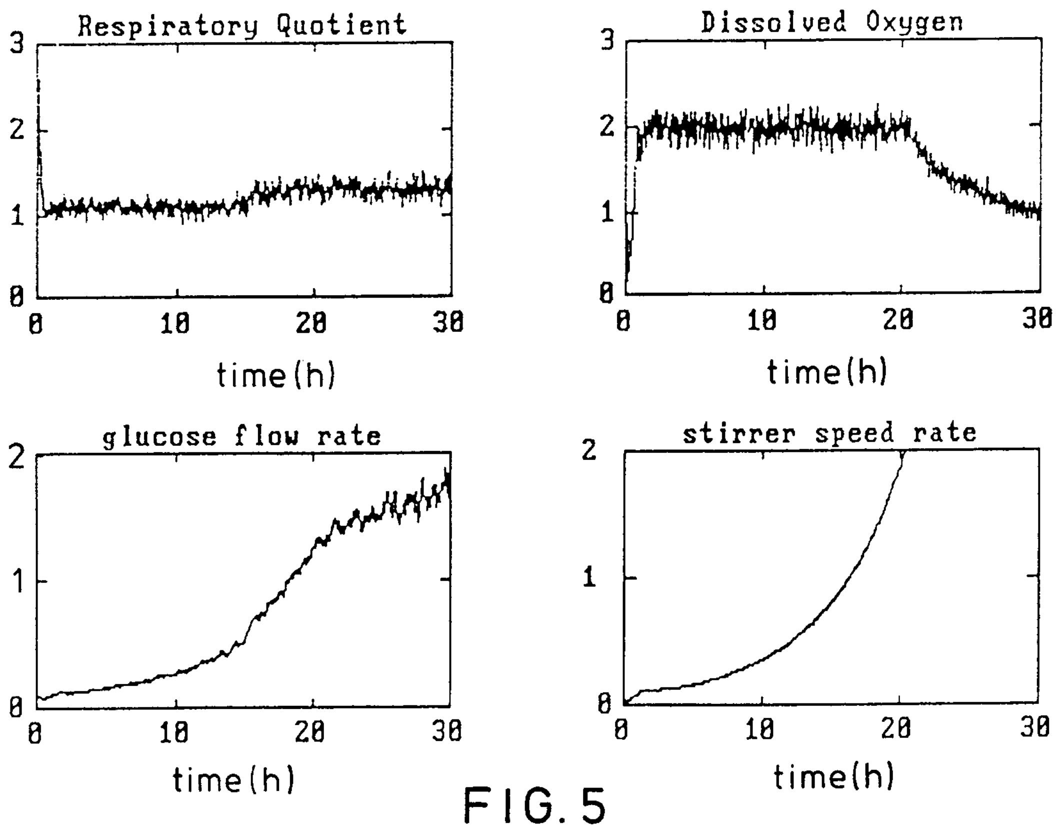
Respiratory Quotient
3
2
1
0
0
10
20
30
time(h)
Dissolved Oxygen
3
2
1
0
0
10
20
30
time(h)
glucose flow rate
2
1
0
0
10
20
30
time(h)
stirrer speed rate
2
1
0
0
10
20
30
time(h)

FIG. 5

European Patent Office

# EUROPEAN SEARCH REPORT

Application Number

EP 91 20 1375

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | PROCEEDINGS OF THE 1985 AMERICAN CONTROL CONFERENCE<br>vol. 2, June 1985, BOSTON US<br>pages 1155 - 1160;<br>A.R. VAN CAUWENBERGHE: 'SELF-ADAPTIVE LONG-RANGE PREDICTIVE CONTROL'<br>* page 1155, right column, line 1 - page 1157, left column, line 10 *<br>--- | 1 | G05B13/04<br>G05B21/02<br>C12M1/00 |
| A | TRANSACTIONS OF THE SOCIETY OF INSTRUMENT AND CONTROL ENGINEERS<br>vol. 27, no. 3, March 1991,<br>pages 255 - 262;<br>T. AKASHI: 'MEASUREMENT OF SPECIFIC GROWTH RATE WITH FRACTAL DIMENSION IN BIOPROCESS'<br>* abstract *<br>--- | 1 | |
| A,D | OPERATIONS RESEARCH<br>vol. 21, 1973,<br>pages 1071 - 1088;<br>R.D. SMALLWOOD & E.J. SONDIK: 'THE OPTIMAL CONTROL OF PARTIALLY OBSERVABLE MARKOV PROCESSES OVER A FINITE HORIZON'<br>* the whole document *<br>--- | 1 | **TECHNICAL FIELDS SEARCHED (Int. Cl.5)** |
| | | | G05B |
| A | JOURNAL OF GUIDANCE AND CONTROL AND DYNAMICS.<br>vol. 12, no. 2, 1989, 1EW YORK US<br>pages 209 - 219;<br>J. MCGOUGH, A. REIBMAN AND K. TRIVEDI: 'MARKOV RELIABILITY MODELS FOR DIGITAL FLIGHT CONTROL SYSTEMS'<br>* paragraph III; figures 1-6 *<br>----- | 2 | |

The present search report has been drawn up for all claims

| Place of search | Date of completion of the search | Examiner |
|---|---|---|
| THE HAGUE | 07 FEBRUARY 1992 | GOETZ P.A. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document